# EUROPÄISCHE PATENTSCHRIFT

(11) **EP 3 213 675 B1**
(45) Veröffentlichungstag und Bekanntmachung des Hinweises auf die Patenterteilung: **26.06.2019**
(21) Anmeldenummer: 17159126.6
(22) Anmeldetag: 03.03.2017
(51) Int. Cl.: A61B 1/05, G01B 9/02, A61B 5/12, A61B 1/00, A61B 5/00, A61B 1/227

(54) **VORRICHTUNG UND SYSTEM ZUR DOPPLER OPTISCHEN KOHÄRENZTOMOGRAFIE (OCT) AM HUMANEN MITTELOHR**
DEVICE AND SYSTEM FOR DOPPLER OPTICAL COHERENCE TOMOGRAPHY (OCT) FOR THE HUMAN MIDDLE EAR
DISPOSITIF ET SYSTEME DE TOMOGRAPHIE PAR COHÉRENCE OPTIQUE (OCT) DOPPLER SUR L'OREILLE MOYENNE HUMAINE

(30) Priorität: 04.03.2016 DE 102016203608
(43) Veröffentlichungstag der Anmeldung: 06.09.2017
(73) Patentinhaber: Technische Universität Dresden, 01069 Dresden (DE)
(72) Erfinder: KOCH, Edmund, 01309 Dresden (DE); ROTTMANN, Pascal, 01099 Dresden (DE); KIRSTEN, Lars, 01705 Freital (DE); BURKHARDT, Anke, 01217 Dresden (DE); MEHNER, Mirko, 09661 Hainichen (DE)
(74) Vertreter: Loock, Jan Pieter

(56) Entgegenhaltungen:
- US-A1- 2005 143 664
- US-A1- 2013 060 131
- US-A1- 2015 148 654
- ANKE BURKHARDT ET AL: "Investigation of the human tympanic membrane oscillation ex vivo by Doppler optical coherence tomography : Investigation of the human tympanic membrane oscillation", JOURNAL OF BIOPHOTONICS, Bd. 7, Nr. 6, 7. Juni 2014 (2014-06-07), Seiten 434-441, XP055388199, DE ISSN: 1864-063X, DOI: 10.1002/jbio.201200186
- KIRSTEN LARS ET AL: "Imaging the tympanic membrane oscillation ex vivo with Doppler optical coherence tomography during simulated Eustachian catarrh", PROGRESS IN BIOMEDICAL OPTICS AND IMAGING, SPIE - INTERNATIONAL SOCIETY FOR OPTICAL ENGINEERING, BELLINGHAM, WA, US, Bd. 9541, 9. Juli 2015 (2015-07-09), Seiten 95410R-95410R, XP060056477, ISSN: 1605-7422, DOI: 10.1117/12.2183620 ISBN: 978-1-5106-0027-0
- BURKHARDT ANKE ET AL: "Endoscopic optical coherence tomography for imaging the tympanic membrane", OPTICAL COHERENCE TOMOGRAPHY AND COHERENCE TECHNIQUES V, SPIE, 1000 20TH ST. BELLINGHAM WA 98225-6705 USA, Bd. 8091, Nr. 1, 9. Juni 2011 (2011-06-09) , Seiten 1-6, XP060015622, DOI: 10.1117/12.889734 [gefunden am 1901-01-01]

## Beschreibung

### Stand der Technik

Die vorliegende Erfindung geht aus von einer Vorrichtung zur medizinischen Untersuchung des humanen Trommelfells.

Aus dem Stand der Technik sind Otoskopie, Tympanometrie, Audiometrie und Laser-Doppler-Vibrometrie (LDV) und die radiologische Bildgebung als Verfahren zur nicht-chirurgischen Untersuchung eines eingeschränkten Hörvermögens eines Patienten bekannt. Die Otoskopie umfasst eine Begutachtung des Trommelfells durch einen behandelnden Arzt. Nachteiligerweise ist dabei jedoch einerseits nur eine Aufsicht auf das Trommelfell möglich und andererseits handelt es sich um eine rein subjektive Bewertung durch den Arzt. Bei der Tympanometrie, der Audiometrie und anderen akustischen Verfahren wird hingegen das Hörvermögen bzw. die Impedanz/Compliance vermessen, wobei allerdings nur integrale Aussagen über das gesamte Mittelohr möglich sind. Eine isolierte Beurteilung allein des Trommelfells ist hingegen nicht oder nur schwer möglich. Die Laser-Doppler-Vibrometrie basiert auf der frequenzaufgelösten Vermessung der Schwingung des Trommelfells, jedoch lediglich an einem einzigen Punkt des Trommelfells. Entsprechende Vorrichtungen zur Durchführung der vorstehend genannten Verfahren sind beispielsweise in den Druckschriften WO 00 / 64 328 A1 und US 2008 / 0 262 314 A1 offenbart.

Zur Verbesserung der frühzeitigen und sicheren Diagnose verschiedener Pathologien, wie Mittelohrentzündung, Paukenerguss, Lokalisierung von Schallleitungsstörungen, ist eine ortsaufgelöste und dreidimensionale Vermessung der Struktur des humanen Trommelfells wünschenswert. Ein Verfahren, mit welchem dieses Ziel erreicht werden kann, ist die optische Kohärenztomografie (engl. optical coherence tomography, OCT). Hierbei handelt es sich um ein aus dem Stand der Technik bekanntes Verfahren, welches eine kontaktlose und dreidimensionale Vermessung der Struktur des humanen Trommelfells ermöglicht, indem Licht einer breitbandigen, kurzkohärenten Lichtquelle mittels eines Strahlteilers in einen Proben- und einen Referenzstrahl aufgeteilt wird und der am zu untersuchenden Trommelfell gestreute Probenstrahl anschließend mit dem an einem Spiegel reflektierten Referenzstrahl überlagert wird. Die interferierenden Strahlen werden spektral aufgelöst detektiert. Beispielsweise wird bei der Fourier domain OCT aus einem Interferenzspektrum via Fouriertransformation ein Tiefenprofil der Reflektivität berechnet. Zur Erzeugung eines dreidimensionalen Abbildes des Trommelfells wird das Trommelfell in die zwei lateralen Richtungen punktweise abgetastet. Ein Endoskop zur Durchführung der optischen Kohärenztomografie ist beispielsweise aus den Druckschriften US 2013 / 0 060 131 A1, US 2009 / 0 185 191 A1 und US 8,594,757 B2 bekannt. Mit der Erweiterung zur Doppler-OCT können dabei auch frequenzaufgelöste Messungen der Trommelfellschwingungen im Rahmen einer in-vivo Untersuchung realisiert werden. Alternativ zur OCT in der Frequenzdomäne kann auch Time Domain OCT oder zur Parallelisierung die Full-Field OCT und weitere Verfahren verwendet werden.

### Offenbarung der Erfindung

Es ist eine Aufgabe der vorliegenden Erfindung, eine Vorrichtung zur Verfügung zu stellen, welche sowohl eine dreidimensionale ortsaufgelöste Vermessung der Struktur des humanen Trommelfells sowie eine ortaufgelöste und frequenzaufgelöste Messung von Trommelfellschwingungen im Rahmen einer endoskopischen nicht-invasiven in-vivo Untersuchung ermöglicht und dabei gleichzeitig auch eine akustische Anregung der Trommelfellschwingung und die Vermessung des Schalldrucks in unmittelbarer Nähe zum Trommelfell erlaubt. Die Vorrichtung soll zudem einfach aufgebaut und kostengünstig realisierbar sein und den aktuellen gesetzlichen Anforderungen an ein Medizinprodukt entsprechen.

Gelöst wird diese Aufgabe mit einer Vorrichtung zur Doppler optischen Kohärenztomografie (Doppler OCT) am humanen Mittelohr, wobei die Vorrichtung eine Endoskopeinheit zur zumindest teilweisen Einführung in den Gehörgang aufweist, wobei in die Endoskopeinheit eine Schallquelle, eine Schallempfangsvorrichtung und eine OCT-Optik integriert sind.

Die erfindungsgemäße Vorrichtung hat gegenüber dem Stand der Technik den Vorteil, dass die Endoskopeinheit sowohl die Schallquelle zur gut konditionierten, direkten akustischen Stimulation des Trommelfells und die Schallmesseinheit zur Messung des Schalldrucks im Raum vor dem Trommelfell aufweist als auch die OCT-Optik, so dass während der definierten Stimulation des Trommelfells durch die Schallquelle eine Doppler optische Kohärenztomografie durchgeführt werden kann. Die Doppler optische Kohärenztomographie ermöglicht dabei eine Vermessung der Schwingung des Trommelfells, indem über die gemessene Dopplerverschiebung auf die Bewegung des Trommelfells zurückgeschlossen wird. Insbesondere kann mittels der Schallempfangsvorrichtung eine Referenzierung der mittels der optischen Kohärenztomografie gemessenen Trommelfellreaktionen auf das Schallsignal erfolgen. Durch die gleichzeitige Vermessung des Schalldrucks vor dem Trommelfell kann hierbei die gemessene Doppler-Verschiebung zudem in eine Phasenbeziehung zur akustischen Stimulation gesetzt werden. Es ist also nicht nur eine dreidimensionale ortsaufgelöste Vermessung der Struktur des humanen Trommelfells mittels der optischen Kohärenztomografie sondern zugleich auch eine ortaufgelöste und frequenzaufgelöste Messung der Trommelfellschwingungen mittels Doppler-OCT realisiert. Zudem ermöglicht die Verwendung von Schallquelle und Schallempfangsvorrichtung einen Vergleich zwischen emittierter und reflektierter Schallleistung. Die sichere und frühzeitige Diagnose von Pathologien, wie bspw. Mittelohrentzündung, Paukenerguss und Schallleitungsstörungen, sowie die Untersuchung von Patienten mit bereits erfolgter Mittelohrrekonstruktion kann somit erheblich verbessert werden. Der behandelnde Arzt benötigt vorteilhafterweise lediglich die eine erfindungsgemäße Vorrichtung, sodass sich die praktische Handhabung für den behandelnden Arzt gegenüber herkömmlichen Endoskopen kaum verändert. Vorteilhafterweise erfolgen die akustische Stimulation und die Schallmessung mit der erfindungsgemäßen Vorrichtung insbesondere unmittelbar vor dem Trommelfell. Der Gehörgang wird dadurch als akustischer Modulator aus der Messkette ausgeschlossen. Der Vorteil dieser Anordnung ist eine klar definierte, direkte Verkopplung von Schallquelle und Schallempfänger (Trommelfell). Die Schallquelle wird typischerweise auch als Schallgeber oder in derartiger Anwendung auch als Hörer bezeichnet.

Die erfindungsgemäße Vorrichtung ist insbesondere ein Endoskop-Vorsatz, der an gegebene OCT-Messköpfe adaptiert wird. Ein solcher OCT-Messkopf ist typischerweise als Teil eines OCT-Systems mit einer OCT-Zentraleinheit verbunden. Das OCT-System umfasst eine Strahlquelle sowie einen Strahlteiler, der von der Strahlquelle ausgestrahltes breitbandiges, kurzkohärentes Licht in einen Probenstrahl und einen Referenzstrahl aufteilt. Der Strahlteiler ist entweder in den OCT-Messkopf integriert oder in der OCT-Zentraleinheit angeordnet. Der OCT-Messkopf umfasst wenigstens eine Möglichkeit zur Strahlablenkung, z. B. mittels Galvanometerscannern. Der Probenstrahl wird von dem OCT-Messkopf in die OCT-Optik der Endoskopeinheit eingekoppelt und somit auf das Trommelfell gelenkt. Der am Trommelfell reflektierte Probenstrahl wird durch die OCT-Optik zurück in den OCT-Messkopf geleitet und im OCT-Messkopf oder in der OCT-Zentraleinheit mit dem an einem Spiegel reflektierten Referenzstrahl überlagert. Die interferierenden Strahlen werden mittels einer Auswerteeinheit ausgewertet. Zur Erzeugung eines dreidimensionalen Abbildes des Trommelfells wird das Trommelfell hierbei punktweise abgetastet. Ein Interferometer kann in unterschiedlicher Art und Weise implementiert sein. Entweder ist das Interferometer, also Referenzarm und Probenarm, in den OCT-Messkopf integriert oder der Strahlteiler des Interferometers (z. B. ein Faserschmelzkoppler) befindet sich in der OCT-Zentraleinheit. Der Probenarm besteht dann aus einer Glasfaser, die zum OCT-Messkopf führt. Für den Messkopf bedeutet dies, dass dieser nicht zwangsläufig das gesamte Interferometer beinhalten muss. Lediglich eine Einheit zur Strahlablenkung ist auf jeden Fall integriert. Alternativ wäre aber auch eine sogenannte "Common-Path-OCT" denkbar, welche ohne ein klassisches Interferometer auskommt, sondern stattdessen ein zusätzliches optisches Element umfasst, an dessen Grenzschicht Licht aus dem Strahl reflektiert wird, welches mit dem am Trommelfell reflektierten Probenstrahl direkt interferiert. Der Referenzarm und der Probenarm fallen somit zumindest teilweise zusammen. Denkbar ist, dass das optische Element, beispielsweise eine Glasplatte, in die OCT-Optik integriert ist oder am trommelfellseitigen Ausgang der OCT-Optik vorgesehen ist. Bei der Doppler-OCT wird zusätzlich die Dopplerverschiebung im reflektierten Probenstrahl, die durch die Streuung des Probenstrahls am schwingenden bzw. bewegten Trommelfell erfolgt, berücksichtigt. Die Datenaufbereitung kann in bekannter Weise im Rahmen einer "Time Domain OCT" (TD-OCT) oder "Fourier Domain OCT" (FD-OCT), insbesondere "Swept Source OCT" (SS-OCT), und mit Erweiterungen der Datenauswertung, z. B. phasenaufgelöste Doppler-OCT und/oder Polarisationssensitive OCT (PS-OCT) und/oder Spektroskopische OCT (SD-OCT) erfolgen. Die Strahlquelle emittiert insbesondere Licht im IR-Bereich. Alternativ wäre aber auch denkbar, dass die Strahlquelle zumindest teilweise Licht im visuellen Bereich emittiert.

Vorteilhafte Ausgestaltungen und Weiterbildungen der Erfindung sind den Unteransprüchen, sowie der Beschreibung unter Bezugnahme auf die Zeichnungen entnehmbar.

Gemäß einer bevorzugten Ausführungsform der vorliegenden Erfindung ist vorgesehen, dass die Vorrichtung einen Basiskörper und bevorzugt einen austauschbaren Anwendungsaufsatz umfasst, wobei besonders bevorzugt der Anwendungsaufsatz einen Basisbereich, der lösbar an dem Basiskörper befestigbar ist, und einen applikationsseitigen Mündungsbereich, der bei der Anwendung der Vorrichtung dem Trommelfell zugewandt ist, aufweist. In vorteilhafter Weise ist die Vorrichtung somit zweiteilig aus Basiskörper und Anwendungsaufsatz ausgeführt. Lediglich der Anwendungsaufsatz gelangt in unmittelbaren Kontakt mit dem Patienten, so dass von Patient zu Patient lediglich der Anwendungsaufsatz gewechselt oder gereinigt bzw. sterilisiert werden muss, um die erforderliche Hygiene einzuhalten. Denkbar ist, dass der Anwendungsaufsatz aus einem sterilisierbaren Material, wie Metall gefertigt ist. Der Anwendungsaufsatz ist als Trichter ausgebildet, welcher zum Halten und Stabilisieren der OCT-Optik, akustischer Leiter und ggf. Lichtleiter vorgesehen ist. Der Basiskörper weist vorzugsweise sämtliche aktive Bauelemente auf, während der Anwendungsaufsatz vorzugsweise ausschließlich passive Bauelemente aufweist.

Gemäß einer weiteren bevorzugten Ausführungsform der vorliegenden Erfindung ist vorgesehen, dass die OCT-Optik dazu ausgebildet ist, einen im Basisbereich eingekoppelten OCT-Probenstrahl zum Mündungsbereich zu leiten und den am Trommelfell reflektierten Referenzstrahl zurück zum Basisbereich zu transportieren. Der Probenstrahl wird entweder von einem externen OCT-Messkopf eingekoppelt oder von einem in den Basiskörper integrierten OCT-Messkopf bereitgestellt. Der reflektierte bzw. gestreute Probenstrahl wird zur Auswertung in den OCT-Messkopf oder zur OCT-Zentraleinheit zurückgeführt. Die OCT-Optik umfasst hierfür bevorzugt ein optisches Linsensystem, besonders bevorzugt eine oder mehrere Gradientenindex (GRIN)-Linsen oder eine oder mehrere Stablinsen. Vorzugsweise umfasst die OCT-Optik eine Kombination aus GRIN-Relaylinsen und GRIN-Objektivlinsen. Dies hat den Vorteil, dass der Probenstrahl aufgeweitet wird und man somit ein breites Blickfeld im vergleichsweise schmalen Gehörgang erzielt. Dies ist ein besonderer Vorteil gegenüber den aus dem Stand der Technik bekannten Verfahren, da die üblichen telezentrischen Scanverfahren aufgrund von mannigfaltigen Gehörgangsgeometrien keinen vollständigen Blick auf das Trommelfell erlauben.

Wenn ein externer OCT-Messkopf Verwendung findet, weist der Basiskörper insbesondere einen Kopplungsbereich zum Ankoppeln der Vorrichtung an den externen OCT-Messkopf auf, wobei der Kopplungsbereich derart ausgebildet ist, dass im gekoppelten Zustand der OCT-Probenstrahl von einem OCT-Scanner des OCT- Messkopfs in die OCT-Optik eingekoppelt wird und der reflektierte OCT-Probenstrahl zurück zum OCT-Scanner geleitet wird. Der OCT-Messkopf weist hierfür eine Strahl-Ablenkeinheit auf.

Die OCT-Optik ist vorzugsweise Teil des Anwendungsaufsatzes. Alternativ wäre aber auch denkbar, dass die OCT-Optik Teil des Basiskörpers ist. Die OCT-Optik wird vorzugsweise ebenfalls zur Otoskopie und insbesondere zur optischen Videoendoskopie verwendet, welche simultan zur (Doppler) OCT-Messung durchgeführt wird. Denkbar ist, dass der reflektierte Probenstrahl im OCT-Messkopf auf einen halbdurchlässigen / dichroitischen Spiegel gelenkt wird, welcher Licht des sichtbaren Spektrums auf eine optische Kamera, beispielsweise einen CCD-Chip oder auf ein Okular lenkt. Alternativ wäre aber auch denkbar, dass die Endoskopeinheit und insbesondere der Anwendungsaufsatz neben der OCT-Optik eine separate optische Bildübertragungseinheit aufweist, welche zur Übertragung von Bildinformationen zur Otoskopie oder optischen Videoendoskopie dient.

Gemäß einer weiteren bevorzugten Ausführungsform der vorliegenden Erfindung ist vorgesehen, dass der Anwendungsaufsatz mit einem Ohrpassstück zum Abdichten des Gehörgangs versehen ist. Das Ohrpassstück umfasst insbesondere einen Einmalartikel, welcher über den Anwendungsaufsatz gestülpt wird. Das Ohrpassstück weist vorzugsweise eine Außenkontur auf, welche zumindest teilweise der Innenkontur des menschlichen Gehörgangs angepasst ist, so dass das Einführen der Vorrichtung in den Gehörgang eines Patienten möglichst einfach und für den Patienten angenehm ist. Das Ohrpassstück dient dabei zur Abdichtung des Gehörgangs, so dass im Gehörgang für die Tympanometrie ein definierter Druck, der vom Umgebungsdruck abweichen kann, einstellbar ist.

Gemäß einer weiteren bevorzugten Ausführungsform der vorliegenden Erfindung ist vorgesehen, dass die Endoskopeinheit eine Lichteinheit aufweist, wobei die Lichteinheit eine Lichtquelle, einen Anschluss zum Anschließen einer externen Lichtquelle und/oder einen Lichtleiter umfasst. Die Lichteinheit umfasst ferner vorzugsweise eine Lichtquelle, die innerhalb des Basiskörpers angeordnet ist, wobei der Lichtleiter das Licht der Lichtquelle zum Mittelohr leitet, so dass das Trommelfell für die Otoskopie ausgeleuchtet wird. Alternativ wäre aber auch denkbar, dass die Lichteinheit eine Lichtquelle umfasst, die unmittelbar im Mündungsbereich angeordnet ist. In diesem Fall würde lediglich die elektrische Stromversorgung in Form eines elektrischen Leiters durch das Anwendungsteil laufen. Die Lichtquelle könnte beispielsweise eine kompakte Leuchtdiode (LED) sein. Ferner wäre auch denkbar, dass die Lichteinheit einen Anschluss zum Anschließen an eine externe Lichtquelle oder eines weiteren Lichtleiter umfasst, wobei der Anschluss vorzugsweise in den Basiskörper integriert ist. Die externe Lichtquelle könnte in den OCT-Messkopf integriert sein, so dass die erfindungsgemäße Vorrichtung selbst keine Lichtquelle umfasst, sondern lediglich das vom Messkopf ausgehende Licht zum Trommelfell leitet. Der Lichtleiter umfasst insbesondere eine Glasfaser oder ein Glasfaserbündel und ist zumindest teilweise in den Anwendungsaufsatz integriert. Die Lichtquelle emittiert insbesondere Licht im sichtbaren Spektrum. Alternativ
könnte auch eine Beleuchtung über die OCT-Optik erfolgen.

Gemäß einer weiteren bevorzugten Ausführungsform der vorliegenden Erfindung ist vorgesehen, dass der Anwendungsaufsatz einen akustischen Hinleiter umfasst, der zum Leiten von Schall von der Schallquelle zum Mündungsbereich vorgesehen ist, wobei der Hinleiter bevorzugt ein Röhrchen und besonders bevorzugt ein Metallröhrchen umfasst. Analog weist der Anwendungsaufsatz vorzugsweise ferner einen akustischen Rückleiter auf, der zum Leiten von Schall vom Mündungsbereich zur Schallempfangsvorrichtung vorgesehen ist, wobei der Rückleiter bevorzugt ein Röhrchen und besonders bevorzugt ein Metallröhrchen umfasst.

Der Hinleiter und/oder der Rückleiter sind entweder innerhalb oder außerhalb des Anwendungsaufsatzes angeordnet. Die Schallquelle ist vorzugsweise im Basiskörper angeordnet. Alternativ wäre aber auch denkbar, dass die Schallquelle direkt im Mündungsbereich des Anwendungsaufsatzes angeordnet ist. Die akustische Schallempfangsvorrichtung umfasst vorzugsweise ein Mikrophon, wobei die akustische Schallempfangsvorrichtung vorzugsweise in den Basiskörper integriert ist, wobei der Schall vom Mündungsbereich durch den Rückleiter zum Mikrophon geleitet wird. Die Schallquelle und die Schallempfangsvorrichtung sind im Basiskörper akustisch voneinander entkoppelt. Denkbar ist, dass die Schallquelle und die Schallempfangsvorrichtung hierfür jeweils in einer Silikontasche gelagert sind, welche jeweils nur über ein schmales Silikonhaltestück mit dem Basiskörper verbunden sind. Alternativ wäre auch denkbar, dass das Mikrophon direkt im Mündungsbereich angeordnet ist. In diesem Fall werden die aufgenommenen akustischen Signale über elektrische Signalleiter zum Basiskörper und insbesondere zum OCT-Messkopf weitergeleitet.

Gemäß einer weiteren bevorzugten Ausführungsform der vorliegenden Erfindung ist vorgesehen, dass die Vorrichtung eine Justiervorrichtung aufweist, welche zur Justierung eines Abstands zwischen dem Kopplungsbereich und der optischen Bildübertragungseinheit vorgesehen ist, wobei die Justiervorrichtung vorzugsweise ein Verstellgetriebe zur Längsverstellung der optischen Bildübertragungseinheit relativ zum Basiskörper umfasst. Die Justiervorrichtung ermöglicht in vorteilhafterweise einen Arbeitsabstand und die Größe des Blickfeldes zu justieren. Hierfür wird mittels der Justiervorrichtung insbesondere der Abstand zwischen der OCT-Optik und dem mit der Endoskopeinheit gekoppelten externen OCT-Messkopf justiert. Denkbar ist, dass mit Hilfe der Justiervorrichtung die OCT-Optik relativ zum Kopplungsbereich bewegbar ist. Der Basiskörper umfasst hierfür ein feststehendes Basisteil, welches den Kopplungsbereich umfasst und einen relativ zum Basisteil mittels der Justiervorrichtung verschiebbaren Justierteil, an welchem die Aufnahme für den Anwendungsaufsatz und damit auch die OCT-Optik befestigt sind. Der Justierteil umfasst in diesem Fall vorzugsweise auch die Schallquelle, die Schallempfangsvorrichtung und einen Teil der Lichteinheit. Die Justiervorrichtung weist ein Verstellgetriebe auf, welche eine relative Drehbewegung zwischen dem Basisteil und einem Teil der Justiervorrichtung in eine translatorische Bewegung des Justierteils gegenüber dem Basisteil umwandelt, wodurch sich insbesondere der Abstand zwischen dem scannerseitigen Ende der Gradientenindex-Linse oder der Stablinse und dem externen OCT-Scanner ändert. Alternativ wäre auch denkbar, dass die Justiervorrichtung in Form einer fokusvariablen Linse ausgebildet ist, die insbesondere zwischen der OCT-Optik und dem OCT-Messkopf angeordnet ist oder Teil der OCT-Optik ist. Die fokusvariable Linse könnte ferner die Objektivlinse (Triplet) im OCT-Messkopf ersetzen.

Da das Trommelfell nicht senkrecht zum Gehörgang steht, weist eine andere Ausführungsform einen Spiegel oder ein Prisma am trommelfellseitigen Ende des Endoskops auf. Solche starren oder einstellbaren Ablenkeinheiten sind z.B. aus den Schriften EP 2514356 A1, EP 1215990 B1 oder Produktinformationen zu Boreskopen der Fa. Karl Storz bekannt.

Gemäß einer weiteren bevorzugten Ausführungsform der vorliegenden Erfindung ist vorgesehen, dass der Basiskörper einen internen OCT-Scanner umfasst, welcher den Probenstrahl in die OCT-Optik einkoppelt und zum Abtasten des Trommelfells vorgesehen ist, und möglicherweise ferner eine Auswerteeinheit zur Durchführung einer Doppler-optischen Kohärenztomographie (Doppler OCT) auf Basis von Bild- und Schallinformationen, die vom OCT-Scanner und vom Mikrophon bereitgestellt werden, aufweist. Vorteilhafterweise wird somit die gesamte Doppler-OCT-Datenauswertung bereits innerhalb der Endoskopeinheit vorgenommen.

Gemäß einer weiteren bevorzugten Ausführungsform der vorliegenden Erfindung ist vorgesehen, dass die Endoskopeinheit einen zusätzlichen Druckkanal aufweist, über welchen der Druck im Bereich des Gehörgangs beeinflusst werden kann. Der Druckkanal ist mit einer Druckquelle verbunden, wobei die Druckquelle vorzugsweise in den Basiskörper oder in den OCT-Messkopf integriert ist. Der Druckkanal kann alternativ vollständig oder teilweise mit einem der akustischen Leiter, dem Hinleiter oder Rückleiter übereinstimmen bzw. zusammenfallen. Der Druckkanal kann intern oder extern verlaufen. Die Druckquelle kann zum Bereitstellen von Unterdruck und/oder Überdruck vorgesehen sein. Um einen definierten Druck im Gehörgang einzustellen, muss der Gehörgang gegenüber dem Umgebungsdruck druckdicht verschlossen werden. Die Endoskopeinheit wird hierfür vorzugsweise mit einem entsprechenden Ohrpassstück oder einem über das Endoskoprohr gestülpten Silikon-Dom versehen.

Ein weiterer Gegenstand der vorliegenden Erfindung ist ein System zur optischen Kohärenztomografie, vorzugsweise Doppler optischen Kohärenztomografie (Doppler OCT), des humanen Trommelfells aufweisend einen OCT-Messkopf und eine mit dem OCT-Messkopf gekoppelte erfindungsgemäße Vorrichtung. Der OCT-Messkopf weist vorzugsweise den OCT-Scanner auf und ist vorzugsweise mit einer OCT-Zentraleinheit verbunden, in welche die Auswerteeinheit zur Durchführung einer Doppler-optischen Kohärenztomographie auf Basis von Bild- und Schallinformationen, die vom OCT-Messkopf und vom Mikrophon bereitgestellt werden, integriert ist.

Weitere Einzelheiten, Merkmale und Vorteile der Erfindung ergeben sich aus den Zeichnungen, sowie aus der nachfolgenden Beschreibung von bevorzugten Ausführungsformen anhand der Zeichnungen. Die Zeichnungen illustrieren dabei lediglich beispielhafte Ausführungsformen der Erfindung, welche den wesentlichen Erfindungsgedanken nicht einschränken.

### Kurze Beschreibung der Zeichnungen

- **Figuren 1 und 2**: zeigen schematische Schnittbildansichten einer Vorrichtung zur Doppler optischen Kohärenztomografie (Doppler OCT) des humanen Trommelfells gemäß einer beispielhaften Ausführungsform der vorliegenden Erfindung.
- **Figur 3**: zeigt eine schematische Ansicht eines Systems zur Doppler optischen Kohärenztomografie (Doppler OCT) des humanen Trommelfells gemäß der beispielhaften Ausführungsform der vorliegenden Erfindung.

### Ausführungsformen der Erfindung

In den verschiedenen Figuren sind gleiche Teile stets mit den gleichen Bezugszeichen versehen und werden daher in der Regel auch jeweils nur einmal benannt bzw. erwähnt.

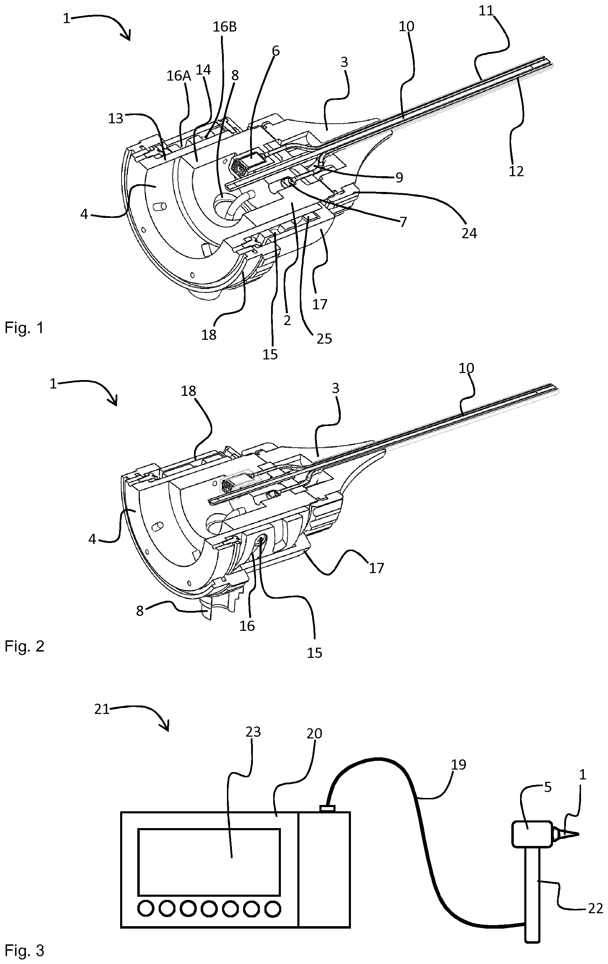

In **Figuren 1 und 2** sind schematische Schnittbildansichten einer Vorrichtung 1 zur Doppler optischen Kohärenztomografie (Doppler OCT) des humanen Trommelfells gemäß einer beispielhaften Ausführungsform der vorliegenden Erfindung dargestellt.

Die Vorrichtung 1 umfasst im vorliegenden Beispiel eine Endoskopeinheit (auch als Endoskop-Vorsatz bezeichnet), welcher auf einen OCT-Messkopf 5 (vgl. Figur 3) eines OCT-Systems 21 aufsetzbar ist. Die Endoskopeinheit umfasst einen Basiskörper 2 und einen auf den Basiskörper 2 lösbar aufgesteckten und mit einer Überwurfmutter 24 gesicherten Anwendungsaufsatz 3. Der Basiskörper 2 umfasst einen Kopplungsbereich 4, mit welchem der Basiskörper 2 an den OCT-Messkopf 5 gekoppelt wird. Ferner sind in den Basiskörper 2 eine Schallquelle 6 sowie eine Schallempfangsvorrichtung 7 in Form eines Mikrophons integriert. Zuletzt weist der Basiskörper 2 einen Anschluss 8 für eine externe Lichtquelle (nicht abgebildet) auf, über welchen Licht der externen Lichtquelle eingekoppelt wird. Die externe Lichtquelle stellt insbesondere Licht im sichtbaren Spektrum bereit.

Der Anwendungsaufsatz 3 umfasst einen Basisbereich, der dem OCT-Messkopf 5 zugewandt ist, und einen Mündungsbereich, der bei der Anwendung dem Trommelfell zugewandt ist. Der Anwendungsaufsatz 3 wird zumindest geringfügig in den Gehörgang eines Patienten eingeführt. Vom Basisbereich zum Mündungsbereich ist der Anwendungsaufsatz 3 zur Erhöhung der Ergonomie stark verjüngt ausgestaltet. Im Inneren des Anwendungsaufsatzes 3 sind mehrere Schall- und Lichtleiter angeordnet, welche durch die Wandung des Anwendungsaufsatzes 3 gestützt und gehalten werden. Im vorliegenden Beispiel ist die Wandung des Anwendungsaufsatzes 3 hierfür aus Metall gefertigt, wodurch gleichzeitig die erforderlichen Hygieneanforderungen sichergestellt werden können. Zudem ist der Anwendungsaufsatz 3 austauschbar und sterilisierbar, so dass von Patient zu Patient entweder ein neuer oder ein frisch sterilisierter Anwendungsaufsatz 3 auf den Basiskörper 3 aufgesetzt werden kann. Der Anwendungsaufsatz 3 umfasst zunächst eine als Lichtleiter fungierende Lichteinheit 9 in Form eines Glasfaserbündels, in welche im Basiskörper das Licht der externen Lichtquelle eingekoppelt wird und zum Mündungsbereich geleitet wird. Das im Mündungsbereich aus der Lichteinheit 9 austretende Licht dient zur Ausleuchtung des Trommelfells. Das reflektierte Licht dient zur Otoskopie des behandelnden Arztes bzw. für optische Videoendoskopie mittels einer Kamera, beispielsweise einer CCD-Kamera.

Ferner weist der Anwendungsaufsatz 3 eine OCT-Optik 10 auf, welche parallel zum Lichtleiter zwischen dem Mündungsbereich und dem Basisbereich verläuft. Die OCT-Optik 10 umfasst eine oder mehrere Linsen bzw. Stablinsen und vorzugsweise eine Gradientenindex-Linse oder eine Kombination aus Gradientenindex-Linsen und weiteren optischen Elementen. Das basisbereichseitige Ende der optischen Bildübertragungseinheit 10 steht bis in den Kopplungsbereich 4 vor, so dass ein Probenstrahl, welcher vom OCT-Messkopf 5 bereitgestellt wird, in die OCT-Optik 10 eingekoppelt werden kann und mittels der OCT-Optik 10 vom Basisbereich zum Mündungsbereich geleitet wird, wo der Probenstrahl aus der OCT-Optik 10 austritt und auf das Trommelfell trifft. Der am Trommelfell gestreute und reflektierte Probenstrahl tritt im Mündungsbereich in die OCT-Optik 10 ein und wird zurück zum Basisbereich und somit zum OCT-Messkopf 5 geleitet. Dort trifft der Probenstrahl auf weitere Komponenten des OCT-Systems 21, in welchem der Probenstrahl mittels eines Interferometers mit einem Referenzstrahl zur Interferenz gebracht wird, um ein Abbild der Struktur des Trommelfells zu erzeugen. Damit vom OCT-System 21 ein ortsaufgelöstes, dreidimensionales Abbild der Struktur des Trommelfells erzeugt werden kann, wird der Probenstrahl systematisch über das Trommelfell bewegt und das Trommelfell mit Hilfe eines OCT-Scanners des OCT-Systems 21 ortsaufgelöst abgetastet. Eine Strahlablenkung zum systematischen Abtasten des Trommelfells könnte bevorzugt im OCT-Messkopf 5 erfolgen oder alternativ im Basiskörper 2 oder im Mündungsbereich angeordnet sein.

Innerhalb des OCT-Messkopfes 5 oder innerhalb des Basiskörpers 2 ist ein halbdurchlässiger Spiegel vorgesehen, welcher Licht im sichbaren Spektrum aus dem reflektierten Probenstrahl herausfiltert und zur Otoskopie auf ein Okular oder zur optischen Videoendoskopie auf eine CCD-Kamera führt. Auf diese Weise kann parallel zur OCT eine klassische optische Otoskopie/Endoskopie durchgeführt werden. Der halbdurchlässige Spiegel und die CCD-Kamera sind optional in den Basiskörper 2 der Endoskopeinheit integriert. Alternativ wäre auch denkbar, dass für die Otoskopie eine zusätzliche Bildübertragungseinheit in dem Anwendungsaufsatz oder eine CCD-Kamera im Mündungsbereich vorgesehen ist.

Der Anwendungsaufsatz 3 weist ferner einen akustischen Hinleiter 11 in Form eines Metallröhrchens auf. Durch den akustischen Hinleiter 11 werden Schallwellen, die von der Schallquelle 6 emittiert werden, zum Mündungsbereich geleitet, um das Trommelfell definiert zu stimulieren. Das in Schwingung versetzte Trommelfell kann nun mittels des OCT-Scanners analysiert werden und dabei mittels der Doppler-OCT eine frequenzaufgelöste und ortsaufgelöste Vermessung der Trommelfellschwingung durchgeführt werden, indem zusätzlich die Dopplerverschiebung im Probenstrahl, die durch die Streuung des Probenstrahls am schwingenden bzw. bewegten Trommelfell erfolgt, berücksichtigt wird. Die Datenaufbereitung kann in bekannter Weise im Rahmen einer "Time Domain OCT" (TD-OCT) oder "Fourier Domain OCT" (FD-OCT), insbesondere "Swept Source OCT" (SS-OCT) erfolgen. Die Endoskopeinheit ermöglicht somit simultan zur OCT und zur Otoskopie ferner eine Doppler-OCT. Ebenso sind Erweiterungen der Datenauswertung, z. B. im Sinne der Polarisationssensitive OCT oder Spektroskopischen OCT (SD-OCT), denkbar.

Die OCT-Optik 10 fungiert als Optik für den OCT-Scanner. Zur Einstellung der optischen Parameter, wie Arbeitsabstand und Größe des Blickfeldes, weist die Endoskopeinheit eine Justiervorrichtung auf, mit welcher der Abstand zwischen dem OCT-Scanner bzw. dem Kopplungsbereich 4 und dem basisbereichseitigem Ende der optischen Bildübertragungseinheit 10 justierbar ist. Hierfür weist der Basiskörper 2 ein Basisteil 13 und ein gegenüber dem Basisteil 13 verschiebbaren Justierteil 14 auf. Der Basisteil 13 umfasst den Kopplungsbereich 4, während das Justierteil 14 mit dem Anwendungsaufsatz 3 verbunden ist. Eine Relativbewegung zwischen Basisteil 13 und Justierteil 14 in Längsrichtung führt somit dazu, dass sich der Abstand zwischen dem OCT-Scanner und der optischen Bildübertragungseinheit 10 ändert. Das Basisteil 13 und das Justierteil 14 sind über ein Verstellgetriebe miteinander gekoppelt. Das Verstellgetriebe umfasst einen äußeren Hohlzylinder 25, in welchen Führungsnuten 16 eingebracht sind. Der Hohlzylinder 25 weist Führungsnuten 16A auf, welche in Längsrichtung keine Steigung aufweisen, also streng in Umfangsrichtung verlaufen. In diesen Führungsnuten 16A laufen wenigstens ein vom Basisteil 13 nach außen radial abstehende Führungsstifte 15, welche Basisteil 13 und Hohlzylinder 25 in Längsrichtung zueinander fixieren und trotzdem eine Drehbewegung zwischen Basisteil 13 und Hohlzylinder 25 zulassen. Der Hohlzylinder 25 weist ferner auch solche Führungsnuten 16B auf, welche sich gewindeartig schräg über den Umfang des Hohlzylinders 25 erstrecken, also nicht nur eine Richtungskomponente in Umfangsrichtung sondern auch eine Richtungskomponente in Längsrichtung aufweisen. In diesen schrägen Führungsnuten 16B laufen zwei vom Justierteil 14 nach außen radial abstehende Führungsstifte (in den Figuren aus perspektivischen Gründen nicht zu sehen), wodurch eine Drehung des Justierteils 14 gegenüber dem Hohlzylinder 25 zu einer Längsverschiebung zwischen Justierteil 14 und Hohlzylinder 25 und somit auch zu einer Längsverschiebung zwischen Justierteil 14 und Basisteil 13 führt. Auf diese Weise kann also der Abstand der OCT-Optik 10 zum OCT-Messkopf 5 justiert werden. Damit die Führungsstifte des Justierteils 14 bis in die Führungsnuten 16B ragen, weist das Basisteil 13, welches in radialer Richtung zwischen dem Justierteil 14 und dem Hohlzylinder 25 angeordnet ist, entsprechende als Langlöcher ausgebildete Aussparungen für die Führungsstifte des Justierteils 14 auf, die zugleich eine Rotation des Justierteils 14 unterbinden. Zur Abschirmung des Verstellgetriebes ist ein Gehäuse 17 vorgesehen, in welches der Hohlzylinder 25 gekapselt ist. Ferner ist ein Verstellring 18 vorgesehen, der mit dem Hohlzylinder 25 verbunden ist, so dass zur Justierung lediglich der Verstellring 18 gegenüber dem Gehäuse 17 durch den Benutzer manuell verdreht werden muss. Der Anschluss 8 für den Lichtleiter 9 ist im Bereich des Justierteils 14 durch das Gehäuse 17 hindurch angeordnet, so dass die Lichtverbindung beim Justieren nicht unterbrochen wird.

Im vorliegenden Beispiel weist der Anwendungsaufsatz 3 zudem einen akustischen Rückleiter 12 auf, welcher vorzugsweise ebenfalls als Metallröhrchen ausgebildet ist und Schallwellen im Bereich des Trommelfells zur Schallempfangsvorrichtung 7 leitet. Mittels der Schallempfangsvorrichtung 7 kann sodann in Verbindung mit einem unter-/überdruckerzeugenden System, welches über einen zusätzlichen Druckkanal eine statische Druckänderung im Gehörgang erzeugt, neben der OCT- und Doppler-OCT-Analyse auch die klassische Tympanometrie durchgeführt werden. Der Druckkanal kann alternativ vollständig oder teilweise mit dem Hinleiter oder Rückleiter übereinstimmen bzw. zusammenfallen und extern oder intern verlaufen. Um den definierten Druck im Gehörgang einzustellen, muss der Gehörgang gegenüber dem Umgebungsdruck druckdicht verschlossen werden. Die Endoskopeinheit wird hierfür vorzugsweise mit einem entsprechenden Ohrpassstück versehen. Ferner kann bei der Doppler-OCT die Phasenbeziehung zwischen der Stimulation des Trommelfells und der durch die Dopplerverschiebung gemessenen Bewegung bzw. Schwingung des Trommelfells bestimmt werden.

Ein System 21 zur Doppler optischen Kohärenztomografie (Doppler OCT) des humanen Trommelfells gemäß der beispielhaften Ausführungsform der vorliegenden Erfindung ist in **Figur 3** illustriert.

Das in Figur 3 gezeigte OCT-System 21 umfasst die in Figuren 1 und 2 illustrierte Vorrichtung 1, sowie einen OCT-Messkopf 5. Der OCT-Messkopf 5 ist dabei in ein Handendoskop eingebettet, welches optional mit einem Griff 22 versehen ist und über eine Leitung 19 mit einer OCT-Zentraleinheit 20 verbunden ist. Die Endoskopeinheit ist über den oben beschriebenen Kopplungsbereich 4 mit dem OCT-Messkopf 5 verbunden. Ein in den OCT-Messkopf 5 integrierter OCT-Scanner kann somit die OCT und Doppler-OCT durchführen. Der OCT-Messkopf 5 weist ferner die Lichtquelle auf, die über den Anschluss 8 in den Lichtleiter eingekoppelt wird.

Die aufgenommenen Daten des OCT-Messkopfs 5 werden digital oder optisch an die Zentraleinheit 20 übermittelt und dort ausgewertet. Denkbar ist, dass die Zentraleinheit 20 eine entsprechende Auswerteeinheit zur Durchführung der Doppler optischen Kohärenztomographie auf Basis von Bild- und Schallinformationen, die vom OCT-Scanner und vom Mikrophon bereitgestellt werden, aufweist. Zudem weist die Zentraleinheit 20 einen Bildschirm 23 auf, in welchem die Ergebnisse der OCT und Doppler-OCT und wahlweise zusätzliche Bildinformationen zu Videoendoskopie/Otoskopie und Tympanometrie angezeigt werden. Die Endoskopeinheit oder die Zentraleinheit 20 sind vorzugsweise mit Signalelementen, wie Signaltongebern oder -leuchten ausgestattet, um dem behandelnden Arzt beispielsweise das Ende des Messvorgangs oder einen bestimmten Betriebszustand bekannt zu machen. Denkbar ist auch, dass der Messkopf bzw. das Endoskop oder der Griff des Endoskops mit Tastern versehen ist, um Messvorgänge zu starten, zu stoppen und dergleichen.

### Bezugszeichenliste

- 1: Vorrichtung
- 2: Basiskörper
- 3: Anwendungsaufsatz
- 4: Kopplungsbereich
- 5: OCT-Messkopf
- 6: Schallquelle
- 7: Schallempfangsvorrichtung
- 8: Anschluss für eine externe Lichtquelle
- 9: Lichteinheit
- 10: OCT Optik
- 11: Hinleiter (akustisch)
- 12: Rückleiter (akustisch)
- 13: Basisteil
- 14: Justierteil
- 15: Führungsstifte
- 16: Führungsnut
- 17: Gehäuse
- 18: Verstellring
- 19: Leitung
- 20: Zentraleinheit
- 21: OCT-System
- 22: Griff / Handstück
- 23: Bildschirm
- 24: Überwurfmutter
- 25: Hohlzylinder

## Patentansprüche

1. Vorrichtung (1) zur Doppler optischen Kohärenztomografie (Doppler OCT), vorzugsweise am humanen Mittelohr, wobei die Vorrichtung (1) eine Endoskopeinheit zur zumindest teilweisen Einführung in den Gehörgang aufweist, wobei in die Endoskopeinheit eine Schallquelle (6), eine Schallempfangsvorrichtung (7) und eine OCT-Optik (10) integriert sind, wobei die Vorrichtung (1) einen Basiskörper (2) und einen Anwendungsaufsatz (3) umfasst, **dadurch gekennzeichnet, dass** der Anwendungsaufsatz (3) einen akustischen Hinleiter umfasst, der zum Leiten von Schall von der Schallquelle (6) zum Mündungsbereich vorgesehen ist.

2. Vorrichtung (1) nach Anspruch 1, wobei der Anwendungsaufsatz (3) austauschbar ist, wobei bevorzugt der Anwendungsaufsatz (3) einen Basisbereich, der lösbar an dem Basiskörper (2) befestigbar ist, und einen Mündungsbereich, der bei der Anwendung der Vorrichtung (1) dem Trommelfell zugewandt ist, aufweist.

3. Vorrichtung (1) nach Anspruch 2, wobei die OCT-Optik (10) dazu ausgebildet ist, einen im Basisbereich eingekoppelten OCT-Probenstrahl zum Mündungsbereich zu leiten und den am Trommelfell reflektierten Probenstrahl zurück zum Basisbereich zu transportieren.

4. Vorrichtung nach einem der vorhergehenden Ansprüche, wobei die OCT-Optik (10) ein optisches Linsensystem, insbesondere eine oder mehrere Gradientenindex-Linsen oder eine oder mehrere Stablinsen, umfasst und wobei die OCT-Optik (10) vorzugsweise Teil des Anwendungsaufsatzes ist.

5. Vorrichtung (1) nach einem der vorhergehenden Ansprüche, wobei der Basiskörper (2) einen Kopplungsbereich (4) zum Ankoppeln an einen externen OCT-Messkopf (5) aufweist, wobei der Kopplungsbereich (4) derart ausgebildet ist, dass im gekoppelten Zustand der OCT-Probenstrahl von dem OCT-Scanner in die OCT-Optik (10) eingekoppelt wird und der reflektierte OCT-Probenstrahl zurück zum OCT-Scanner transportiert wird.

6. Vorrichtung (1) nach einem der vorhergehenden Ansprüche, wobei die Endoskopeinheit eine Lichteinheit (9) aufweist, wobei die Lichteinheit (9) eine Lichtquelle und/oder einen Anschluss (8) zum Anschließen einer externen Lichtquelle und/oder einen Lichtleiter umfasst.

7. Vorrichtung nach Anspruch 6, wobei der Lichtleiter insbesondere eine Glasfaser oder ein Glasfaserbündel umfasst und in den Anwendungsaufsatz (3) und/oder in den Basiskörper integriert ist und/oder wobei der Anschluss (8) vorzugsweise in den Basiskörper (2) integriert ist.

8. Vorrichtung (1) nach einem der vorhergehenden Ansprüche, wobei der Hinleiter ein Röhrchen, bevorzugt ein Metallröhrchen, umfasst.

9. Vorrichtung (1) nach einem der vorhergehenden Ansprüche, wobei der Anwendungsaufsatz (3) einen akustischen Rückleiter umfasst, der zum Leiten von Schall vom Mündungsbereich zur Schallempfangsvorrichtung (7) vorgesehen ist, wobei der Rückleiter bevorzugt ein Röhrchen und besonders bevorzugt ein Metallröhrchen umfasst.

10. Vorrichtung (1) nach einem der vorhergehenden Ansprüche, wobei die akustische Schallempfangsvorrichtung (7) in den Basiskörper (2) integriert ist und vorzugsweise ein Mikrophon umfasst.

11. Vorrichtung (1) nach einem der vorhergehenden Ansprüche, wobei die Schallquelle (6) in den Basiskörper (2) integriert ist.

12. Vorrichtung (1) nach einem der vorhergehenden Ansprüche, wobei der Anwendungsaufsatz (3) mit einem Ohrpassstück zum Abdichten des Gehörgangs versehen ist.

13. Vorrichtung (1) nach einem der vorhergehenden Ansprüche, wobei der Basiskörper (2) eine Justiervorrichtung aufweist, welche zur Justierung eines Abstands zwischen dem Kopplungsbereich (4) und der optischen Bildübertragungseinheit (10) vorgesehen ist, wobei die Justiervorrichtung vorzugsweise ein Verstellgetriebe zur Längsverstellung der optischen Bildübertragungseinheit (10) relativ zum Kopplungsbereich (4) umfasst.

14. Vorrichtung (1) nach einem der vorhergehenden Ansprüche, wobei der Basiskörper (2) einen internen OCT-Messkopf (5) umfasst.

15. Vorrichtung (1) nach Anspruch 14, wobei die Vorrichtung (1) ferner eine Auswerteeinheit zur Durchführung einer Doppler optischen Kohärenztomographie (Doppler OCT) auf Basis von Bild- und Schallinformationen, die vom OCT-Scanner und vom Mikrophon bereitgestellt werden, aufweist.

16. System (21) zur Doppler optischen Kohärenztomografie (Doppler OCT) des humanen Trommelfells aufweisend einen OCT-Messkopf (5) und eine mit dem OCT-Messkopf (5) gekoppelte Vorrichtung (1) nach einem der Ansprüche 1 bis 15.

## Claims

1. Device (1) for Doppler optical coherence tomography (Doppler OCT), preferably on the human middle ear, the device (1) having an endoscope unit for at least partial insertion into the auditory canal, a sound source (6), a sound-receiving device (7) and OCT optics (10) being integrated into the endoscope unit, the device (1) comprising a base body (2) and an application fixture (3), **characterised in that** the application fixture (3) comprises an acoustic output guide which is provided for passing sound from the sound source (6) to the opening region.

2. Device (1) according to claim 1, wherein the application fixture (3) is replaceable, the application fixture (3) preferably having a base region, which is detachably fixable to the base body (2), and an opening region, which faces the eardrum during application of the device (1).

3. Device (1) according to claim 2, wherein the OCT optics (10) are formed to transport an OCT sample stream inputted in the base region to the opening region and to transport the sample stream reflected on the eardrum back to the base region.

4. Device according to any of the preceding claims, wherein the OCT optics (10) comprise an optical lens system, in particular one or more gradient index lenses or one or more rod lenses, and wherein the OCT optics (10) are preferably part of the application fixture.

5. Device (1) according to any of the preceding claims, wherein the base body (2) has a coupling region (4) for coupling to an external OCT measurement head (5), the coupling head (4) being formed in such a way that in the coupled state the OCT sample stream is inputted to the OCT optics (10) from the OCT scanner and the reflected OCT sample stream is transported back to the OCT scanner.

6. Device (1) according to any of the preceding claims, wherein the endoscope unit has a light unit (9), the light unit (9) comprising a light source and/or a terminal (8) for connecting an external light source and/or a light guide.

7. Device according to claim 6, wherein the light guide in particular comprises a glass fibre or a glass fibre bundle and is integrated into the application fixture (3) and/or into the base body and/or wherein the terminal (8) is preferably integrated into the base body (2).

8. Device (1) according to any of the preceding claims, wherein the output guide comprises a tubule, preferably a metal tubule.

9. Device (1) according to any of the preceding claims, wherein the application fixture (3) comprises an acoustic return guide, which is provided for passing sound from the opening region to the sound-receiving device (7), the return guide preferably comprising a tubule and particularly preferably comprising a metal tubule.

10. Device (1) according to any of the preceding claims, wherein the acoustic sound-receiving device (7) is integrated into the base body (2) and preferably comprises a microphone.

11. Device (1) according to any of the preceding claims, wherein the sound source (6) is integrated into the base body (2).

12. Device (1) according to any of the preceding claims, wherein the application fixture (3) is provided with an ear fitting piece for sealing the auditory canal.

13. Device (1) according to any of the preceding claims, wherein the base body (2) has an adjustment device which is provided for adjusting the distance between the coupling region (4) and the optical image transmission unit (10), the adjustment device preferably comprising a shifting transmission for longitudinally shifting the optical image transmission unit (10) relative to the coupling region (4).

14. Device (1) according to any of the preceding claims, wherein the base body (2) comprises an internal OCT measurement head (5).

15. Device (1) according to claim 14, wherein the device (1) further has an evaluation unit for carrying out Doppler optical coherence tomography (Doppler OCT) on the basis of image and sound information provided by the OCT scanner and the microphone.

16. System (21) for Doppler optical coherence tomography (Doppler OCT) of the human eardrum, having an OCT measurement head (5) and a device (1) according to any of claims 1 to 15 coupled to the OCT measurement head (5).

## Revendications

1. Dispositif (1) de tomographie par cohérence optique Doppler (OCT Doppler), de préférence pour l'oreille moyenne humaine, le dispositif (1) comportant une unité d'endoscope destinée à être au moins en partie introduite dans le conduit auditif, une source sonore (6), un dispositif de réception sonore (7) et un système optique OCT (10) étant intégrés dans l'unité d'endoscope, le dispositif (1) comprenant un corps de base (2) et un embout d'utilisation (3), **caractérisé en ce que** l'embout d'utilisation (3) comprend un conducteur d'amenée acoustique, qui est prévu pour conduire le son de la source sonore (6) à la zone d'ouverture.

2. Dispositif (1) selon la revendication 1, dans lequel l'embout d'utilisation (3) peut être échangé, l'embout d'utilisation (3) comportant de préférence une zone de base, qui peut être fixée de manière détachable au corps de base (2), et une zone d'ouverture, qui est orientée vers le tympan lors de l'utilisation du dispositif (1).

3. Dispositif (1) selon la revendication 2, dans lequel le système optique OCT (10) est conçu pour conduire un faisceau d'échantillonnage OCT injecté dans la zone de base jusqu'à la zone d'ouverture et transporter en retour dans la zone de base le faisceau d'échantillonnage réfléchi sur le tympan.

4. Dispositif selon l'une des revendications précédentes, dans lequel le système optique OCT (10) comprend un système optique de lentilles, en particulier une ou plusieurs lentilles à gradient d'indice ou une ou plusieurs lentilles cylindriques, et dans lequel le système optique OCT (10) fait de préférence partie de l'embout d'utilisation.

5. Dispositif (1) selon l'une des revendications précédentes, dans lequel le corps de base (2) comporte une zone de couplage (4) destinée au couplage avec une tête de mesure OCT (5) externe, la zone de couplage (4) étant conçue de telle manière que, dans l'état couplé, le faisceau d'échantillonnage OCT est injecté du scanner OCT au système optique OCT (10) et le faisceau d'échantillonnage OCT réfléchi est transporté en retour au scanner OCT.

6. Dispositif (1) selon l'une des revendications précédentes, dans lequel l'unité d'endoscope comporte une unité d'éclairage (9), l'unité d'éclairage (9) comprenant une source lumineuse et/ou un raccord (8) destiné au raccordement d'une source lumineuse externe et/ou un conducteur optique.

7. Dispositif selon la revendication 6, dans lequel le conducteur optique comprend en particulier une fibre de verre ou un faisceau de fibres de verre et est intégré dans l'embout d'utilisation (3) et/ou dans le corps de base et/ou le raccord (8) étant de préférence intégré dans le corps de base (2).

8. Dispositif (1) selon l'une des revendications précédentes, dans lequel le conducteur d'amenée comprend un petit tube, de préférence un petit tube métallique.

9. Dispositif (1) selon l'une des revendications précédentes, dans lequel l'embout d'utilisation (3) comprend un conducteur de retour acoustique, qui est prévu pour conduire le son de la zone d'ouverture au dispositif de réception sonore (7), le conducteur de retour comprenant de préférence un petit tube et de manière particulièrement préférée un petit tube métallique.

10. Dispositif (1) selon l'une des revendications précédentes, dans lequel le dispositif de réception sonore (7) acoustique est intégré dans le corps de base (2) et comprend de préférence un microphone.

11. Dispositif (1) selon l'une des revendications précédentes, dans lequel la source sonore (6) est intégrée dans le corps de base (2).

12. Dispositif (1) selon l'une des revendications précédentes, dans lequel l'embout d'utilisation (3) est pourvu d'un embout auriculaire destiné à boucher le conduit auditif.

13. Dispositif (1) selon l'une des revendications précédentes, dans lequel le corps de base (2) comporte un dispositif d'ajustage, qui est prévu pour ajuster un écart entre la zone de couplage (4) et l'unité optique de transmission d'images (10), le dispositif d'ajustage comprenant de préférence un mécanisme de réglage destiné au réglage longitudinal de l'unité optique de transmission d'images (10) par rapport à la zone de couplage (4).

14. Dispositif (1) selon l'une des revendications précédentes, dans lequel le corps de base (2) comprend une tête de mesure OCT (5) interne.

15. Dispositif (1) selon la revendication 14, dans lequel le dispositif (1) comporte en outre une unité d'évaluation destinée à effectuer une tomographie par cohérence optique Doppler (OCT Doppler) sur la base d'informations sous forme d'images et d'informations sonores, qui sont mises à disposition par le scanner OCT et le microphone.

16. Système (21) de tomographie par cohérence optique Doppler (OCT Doppler) du tympan humain, comportant une tête de mesure OCT (5) et un dispositif (1) couplé à la tête de mesure OCT (5) selon l'une des revendications 1 à 15.
